# EUROPEAN PATENT APPLICATION

(11) **EP 4 080 212 A2**
(43) Date of publication of application: **26.10.2022**
(21) Application number: 20901022.2
(22) Date of filing: 16.12.2020
(51) Int. Cl.: G01N 33/543, G01N 33/558, B01L 3/00, G01N 33/68

(54) **IMMUNOCHROMATOGRAPHIC STRIP AND KIT, AND COMPETITIVE IMMUNOCHROMATOGRAPHIC ANALYSIS METHOD USING SAME**

(30) Priority: 16.12.2019 KR 20190168246
(71) Applicant: Proteometech Inc., Seoul 07528 (KR)
(72) Inventor: KIM, Jung Yean, Gimpo-si, Gyeonggi-do 10084 (KR); CHOI, Dong Seob, Siheung-si, Gyeonggi-do 14998 (KR); AN, Jun Yop, Seoul 08335 (KR); LIM, Kook Jin, Seoul 06291 (KR); KIM, Beom Jong, Yeoju-si, Gyeonggi-do 12650 (KR)
(74) Representative: J A Kemp LLP
(86) International application number: PCT/KR2020/018443
(87) International publication number: WO 2021/125785

(57) **Abstract**

The present invention provides an immunochromatographic strip, comprising: a sample pad for accommodating a liquid sample; a conjugate pad connected to one side of the sample pad and accommodating a labeling substance and a target substance analog; a detection pad connected to one side of the conjugate pad and comprising a test line, a first control line, and a second control line; and an absorbent pad connected to one side of the detection pad and receiving liquid remaining after reaction, wherein the test line comprises a first ligand, the first ligand may react with the target substance and a first conjugate, the first control line comprises ligand 2-1 that reacts with the second conjugate, and the second control line comprises ligand 2-2 reacting with the second conjugate, wherein the concentrations of ligand 2-1 and ligand 2-2 are different from each other so that qualitative and quantitative analysis of the target substance in the sample can be conveniently performed.

## Description

### CROSS-REFERENCE TO RELATED APPLICATION

This application claims priority to and the benefit of Korean Patent Application No. 10-2019-0168246, filed on DEC 16, 2019, the disclosure of which is incorporated herein by reference in its entirety.

### BACKGROUND

### 1. Field of the Invention

The present invention relates to an immunochromatographic strip provided with a test line using a competitive immunoassay reaction, a diagnostic kit provided with the immunochromatographic strip and a method for qualitatively and quantitatively analyzing a target substance in a sample, and more specifically, the present invention relates to a method for qualitatively or quantitatively analyzing immunoglobulin G (IgG) in a colostrum sample using a diagnostic kit provided with the immunochromatographic strip.

### 2. Discussion of Related Art

An immunochromatographic analysis method known as a rapid test method is a method capable of qualitatively and quantitatively analyzing a trace amount of a target substance (analyte) in a short period of time using an antigen-antibody reaction, and is used in various fields such as diagnosis or testing of various diseases, medicine, agriculture, the livestock industry, food, the military, and the environment. In such an immunochromatographic analysis, an assay strip including a reactant that can react with a target substance to be detected to show a change or an analyzer in the form of a device in which the assay strip is mounted in a plastic case is generally used.

A typical assay strip includes a sample pad for accommodating a liquid sample; a conjugate pad containing a conjugate in which a labeling substance (for example, a gold particle) that generates a signal that can be sensed by the naked eye or using a sensor is conjugated to a ligand such as an antigen or antibody; a detection pad in which a test line that immobilizes a target substance in the sample and/or a binder (antibody or antigen) that specifically binds to the conjugate and a control line capable of confirming the development of the sample are formed; and an absorbent pad for finally accommodating the liquid sample, and such functional pads are connected in a partially overlapped form in the order listed above, attached to a solid support, and continuously arranged. When the assay strip is mounted and used inside the plastic case, a sample inlet for dropping the sample on the sample pad is formed on the upper part of the case, and a result confirmation window for confirming a test result is formed at a position where the binder of the detection pad is immobilized. As described above, in the immunochromatographic analysis method using the assay strip, when a liquid sample is dropped onto the sample pad, the liquid sample moves through the conjugate pad and the detection pad by capillary action and is finally accommodated in the absorbent pad. For example, an antibody capable of binding to an antigen to be measured is immobilized on a membrane pad in a line form (test line), and another antibody is bound to a labeling substance such as gold particles and develops a sample. The developed sample passes through a conjugate pad and is partially developed by forming a complex of antigen-gold particle-antibody, and the antigen-gold particle-antibody is captured by an immobilized test line to form a 'test line-immobilized antibody-antigen-gold particle-antibody'. As a result, the test line appears to be red due to the gold particles. As described above, the conjugate contained in the conjugate pad also moves with the liquid sample, and when there is a substance to be analyzed in the sample, the conjugate binds to the binder immobilized on the detection pad via the target substance (typically referred to as a "sandwich reaction"). However, when analyzed by a sandwich immunoassay method, the antibody or ligand needs to have two or more binding sites (epitopes) such that the target substance binds to a first-binding antibody and second-binding antibody-labeling substance to show a sandwich reaction, and when the size of the target substance is small (peptide protein, drug, allergen, and the like), a problem of binding affinity due to steric hindrance, and the like and problems of specificity, sensitivity and cross-reactivity due to an animal of origin (host) in which the antibody is produced may occur.

An assay strip based on the aforementioned principles may be used to sense the presence or absence of a target substance in a sample with the naked eye or using a sensor. However, although a rapid kit using such competitive reactions have the advantages of easy mass production application, an easy usage method and fast result interpretation, and low cost without the need for expensive and large specialized analytical equipment, there is a disadvantage in that the quantitativeness is insufficient because the detection limit is relatively low and a visual judgment is generally made. Further, since it is difficult to measure an accurate amount of target substance due to the Hook effect in the quantification of a high concentration of target substance, multiple dilutions or high dilution factors need to be used. For this reason, immunochromatographic analysis methods in the related art are mainly used for qualitative analysis, and have a problem in that there are many limitations in quantitative analysis.

### SUMMARY OF THE INVENTION

A technical problem to be solved by the present invention is to provide a chromatographic strip capable of quantifying and qualitatively analyzing a target substance in a sample with only one test line.

Specifically, a technical problem to be solved by the present invention is to provide an immunochromatographic strip capable of not only qualitatively analyzing a target substance in a sample, but also quantitatively analyzing the target substance with high reliability with the naked eye or using a sensor, and the like, provided with only one test line which causes a direct competitive reaction with the target substance in the sample, but enables any one to rapidly, easily and accurately quantify a high concentration of the target substance with a predetermined amount of sample.

Another technical problem to be solved is to provide a diagnostic kit including the immunochromatographic strip.

Another object of the present invention is to provide a qualitative and quantitative analysis of a target substance in a sample using the immunochromatographic strip or diagnostic kit.

To solve the above-described problems, the present invention provides an immunochromatographic strip, comprising: a sample pad for accommodating a liquid sample; a conjugate pad connected to one side of the sample pad and accommodating a labeling substance and a target substance analog; a detection pad connected to one side of the conjugate pad and comprising a test line, a first control line, and a second control line; and an absorbent pad connected to one side of the detection pad and receiving liquid remaining after a reaction, wherein the test line comprises a first ligand that may react with the target substance or a first conjugate, the first control line comprises ligand 2-1 that reacts with the second conjugate, and the second control line comprises ligand 2-2 that reacts with the second conjugate, wherein the concentrations of ligand 2-1 and ligand 2-2 are different from each other.

Specifically, the present invention provides an immunochromatographic strip in which a concentration measurement range (dynamic range) of a target substance in a sample is 5 mg/ml to 140 mg/ml.

The present invention provides an immunochromatographic strip, in which the first conjugate of the present invention consists of a combination of a target substance analog and a target substance, the first conjugate in a mobile phase and the target substance in the sample competitively react with a first ligand of a test line, a first complex may be formed when the first conjugate reacts with the first ligand of the test line, and the number of first complexes is decreased as the concentration of the target substance in the sample is increased.

Specifically, the present invention provides an immunochromatographic strip in which the first complex is captured by a test line and shows a signal, and the amount of target substance in the sample is measured using the signal.

In the immunochromatographic strip of the present invention, the signal intensity of the test line may be decreased as the concentration of the target substance in the sample is increased.

Further, in the immunochromatographic strip of the present invention, the first ligand, ligand 2-1 and ligand 2-2 include any one selected from the group consisting of a protein, an antigen, an antibody, DNA, RNA, PNA and an aptamer, and the first ligand may include a ligand different from ligand 2-1 and ligand 2-2.

In addition, the present invention provides an immunochromatographic strip in which the labeling substance includes at least one selected from the group consisting of colloidal gold, latex particles, colored polystyrene microparticles, enzymes, fluorescent dyes, conductive polymers, luminous substances and magnetic particles.

According to an exemplary embodiment of the present invention, provided is a diagnostic kit in which an immunochromatographic strip is fixed in a case, wherein the case includes an upper case and a lower case, the lower case includes a strip support, and the upper case includes a sample inlet and a result confirmation window at a position corresponding to a test line or a control line.

Furthermore, provided is a diagnostic kit in which the support includes at least one selected from the group consisting of nitrocellulose, nylon, PVDF, glass and plastic.

According to another exemplary embodiment of the present invention, it is possible to provide an immunochromatographic analysis method, including the steps of: injecting a predetermined liquid including a sample into a sample inlet; forming a mobile phase by accommodating the liquid in a sample pad; allowing the mobile phase to be developed into a conjugate pad and include a first conjugate; allowing the first conjugate and the target substance in the sample to competitively bind to a first ligand while the mobile phase including the first conjugate is developed into a test line; allowing the first conjugate to bind to the first ligand to form a first complex and allowing the formed first complex to show a predetermined first signal according to a labeling substance; allowing the mobile phase to be developed into a first control line on which ligand 2-1 is immobilized and a second control line on which ligand 2-2 is immobilized; showing a predetermined second signal in the first control line and the second control line by allowing a second conjugate to bind to ligand 2-1 and ligand 2-2 whose concentrations are different from each other; and analyzing the amount of the target substance in the sample according to the first signal and the second signal.

### BRIEF DESCRIPTION OF THE DRAWINGS

The above and other objects, features and advantages of the present invention will become more apparent to those of ordinary skill in the art by describing in detail exemplary embodiments thereof with reference to the accompanying drawings, in which:
FIG. 1 is a schematic view illustrating the cross-section of an analysis strip used for a typical immunochromatographic analysis;
FIG. 2 is a schematic view illustrating an analysis strip used in the immunochromatographic analysis of the present invention;
FIG. 3 is a schematic view of a method capable of deriving the results of the immunochromatographic strip according to an exemplary embodiment of the present invention;
FIG. 4 is a schematic view illustrating the pattern of a test line and an invariant control line before introducing a sample of a dipping test method in the immunochromatographic strip of the present invention;
FIG. 5 is a schematic view illustrating the pattern of a test line and an invariant control line after introducing a sample of a dipping test method in the immunochromatographic strip of the present invention;
FIG. 6 illustrates the results of an actual strip showing a comparison of quantitative analysis results for the concentrations of target substances in various samples according to an exemplary embodiment of the present invention; and
FIG. 7 is a quantitative graph showing the intensity of each signal depending on the concentration of the target substances of a plurality of test lines shown by the experimental results of the present invention, and illustrates the quantitative measurement range.

### DETAILED DESCRIPTION OF EXEMPLARY EMBODIMENTS

Hereinafter, the present invention will be described in detail with reference to the accompanying drawings. The benefits and features of the present invention, and the methods of achieving the benefits and features will become apparent with reference to embodiments to be described below. However, the present invention is not limited to the embodiments to be disclosed below, but may be implemented in various other forms, and the present embodiments are only provided for rendering the disclosure of the present invention complete and for fully representing the scope of the invention to a person with ordinary skill in the technical field to which the present invention pertains, and the present invention will be defined only by the scope of the claims. Further, terms such as first and second may be used to describe various constituent elements, but the constituent elements should not be limited by the terms. The terms are used only to distinguish one constituent element from another constituent element. In addition, when it is determined that the detailed description of the related publicly known art in describing the present invention may obscure the gist of the present invention, the detailed description thereof will be omitted.

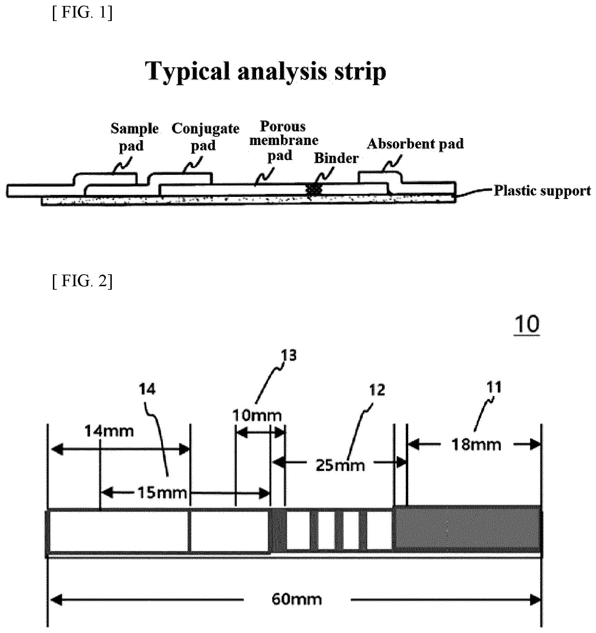

FIG. 1 is a schematic view illustrating the cross-section of an analysis strip used for a typical immunochromatographic analysis, and FIG. 2 is a schematic view illustrating an analysis strip used in the immunochromatographic analysis of the present invention.

Throughout the drawings, the same reference numerals will be used for parts that having similar functions and actions. The present invention relates to an immunochromatographic strip 10 as illustrated in FIG. 1, and may include an absorbent pad 11, a detection pad 12, a conjugate pad 13 and a sample pad 14.

Referring to FIGS. 1 and 2, an immunochromatographic strip 10 of the present invention includes a sample pad 14 for accommodating a liquid sample; a conjugate pad 13 connected to one side of the sample pad and accommodating a labeling substance and a target substance analog; a detection pad 12 connected to one side of the conjugate pad 13 and including a test line, a first control line, and a second control line; and an absorbent pad 11 connected to one side of the detection pad 12 and receiving liquid remaining after a reaction, wherein the test line includes a first ligand, the first ligand may react with the target substance or a first conjugate, the first control line includes ligand 2-1 that reacts with the second conjugate, and the second control line includes ligand 2-2 reacting with the second conjugate, wherein the concentrations of ligand 2-1 and ligand 2-2 are different from each other.

Specifically, as one aspect for achieving the aforementioned objects of quantifying a high concentration of target substance (bovine IgG) in colostrum, the present invention provides, in the immunochromatographic strip of the present invention provided with a conjugate pad and a detection pad, a conjugate pad including a predetermined amount of target substance analog that competes with a target substance and a signal detection labeling substance that binds to the analog, and the analog and the labeling substance are connected to each other before or after the development of a sample to form a first conjugate. When the sample is developed, the first conjugate may move to a detection pad, and the detection pad is provided with a test line and a control line which are separated, a first ligand reacting with a target substance in the sample is immobilized in the test line, and such a test line is formed of one line. The control line includes a second ligand which does not react with the first conjugate or the target substance in the sample and reacts with a predetermined substance (for example, a second conjugate to which the target substance binds), thereby providing an immunochromatographic strip configured such that a binding reaction with the second conjugate occurs. In particular, the control line in the present invention may include a first control line and a second control line, ligand 2-1 and ligand 2-2 may be immobilized on the first control line and the second control line, respectively, and the concentrations of ligand 2-1 and ligand 2-2 are different from each other so that the binding ratio of the second conjugate may be shown differently. Therefore, the signal intensity according to the binding of the second conjugate to the second ligand is shown differently for each control line, so that the concentration of the target substance may be analyzed using the same.

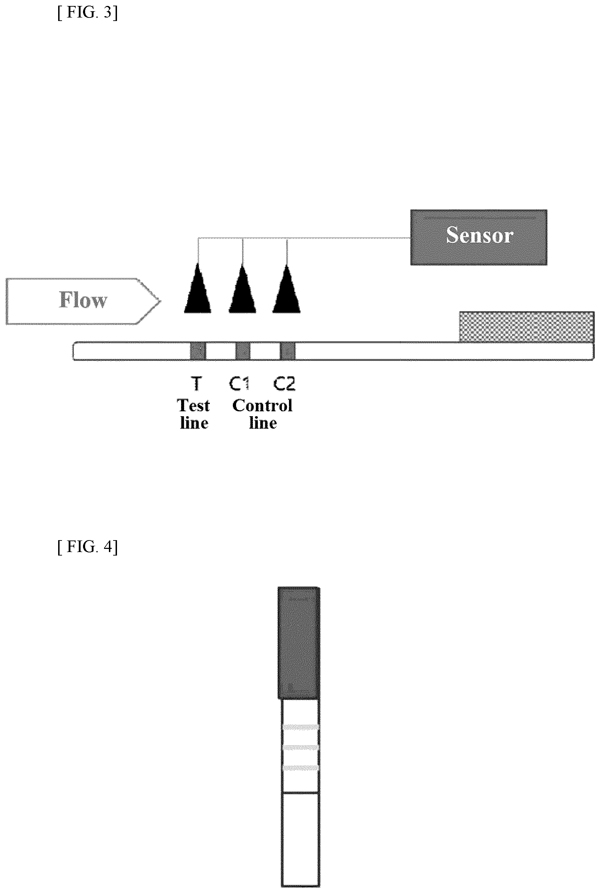

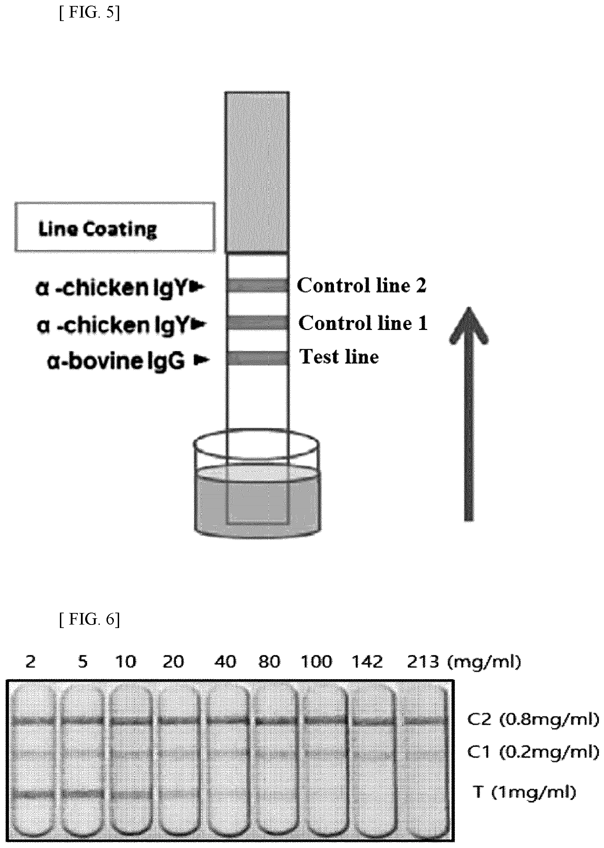

FIG. 4 is a schematic view illustrating the pattern of a test line and an invariant control line before introducing a sample in the immunochromatographic strip of the present invention, and FIG. 5 is a schematic view illustrating the pattern of a test line and an invariant control line after introducing a sample in the immunochromatographic strip of the present invention. Referring to FIGS. 4 and 5, it can be seen that when the immunochromatographic strip of the present invention is used, the target substance can be quantitatively and qualitatively analyzed with the naked eye.

Unlike an immunochromatographic strip in the related art, which is provided with a test line on which an antibody, which detects a material which is the same as the target substance or an analog, is immobilized and a control line capable of confirming the development of the sample by an immune response system which does not affect the development, the immunochromatographic strip of the present invention may include two control lines in which the concentrations of a ligand (second ligand) capable of recognizing a predetermined substance are different from each other. Therefore, it is characterized by using a 'direct competitive immune response system' capable of performing a very reliable quantitative analysis as well as a qualitative analysis of the target substance in the sample by comparing the signals of the two control lines showing a constant signal with a test line showing a target substance with the naked eye or using a predetermined sensor. In particular, the present invention is characterized in that anyone can perform a very reliable semi-quantitative analysis with the naked eye using only two control lines consisting of concentration compartments without the need for using a special analytical instrument.

As used herein, the term "immunochromatography" refers to an analysis method in which an immune response principle based on an antigen-antibody reaction and a chromatography principle in which a sample and a reagent move along a medium by a mobile phase are combined. Briefly, in the analysis method, a reaction with an antigen or antibody to be analyzed in blood is observed by aliquoting the antibody or antigen on a porous membrane in advance to immobilize the antibody or antigen and developing blood from one end of the membrane toward the immobilized antibody or antigen. Although an immune response in a general sense means an antigen-antibody reaction, in the present invention, the immune response includes a reaction between a receptor that specifically binds to a ligand and a ligand that specifically binds thereto, in addition to the antigen-antibody reaction in a broad sense, and is not limited thereto, and may include all reactions that occur by specifically recognizing each other, such as a reaction between an enzyme and a substrate.

Analysis by immunochromatography is performed while the sample including the target substance moves along with the mobile phase by capillarity action through the medium. Therefore, a strip can be manufactured and used as a medium for such immunochromatographic development. The specific components of such an immunochromatographic strip and respective functions thereof will be described below.

According to an exemplary embodiment of the present invention, it is possible to provide an immunochromatographic strip, in which the first conjugate of the present invention consists of a combination of a target substance analog and a target substance, the first conjugate in a mobile phase and the target substance in the sample competitively react with a first ligand of a test line, a first complex may be formed when the first conjugate reacts with the first ligand of the test line, and the number of first complexes is decreased as the concentration of the target substance in the sample is increased, the first complex is captured by the test line and shows a signal, and the amount of the target substance in the sample may be measured using the signal.

Further, according to another exemplary embodiment of the present invention, in the immunochromatographic strip of the present invention, the signal intensity of the test line may be decreased as the concentration of the target substance in the sample is increased.

A labeling substance may be used to readily confirm the reaction between the target substance or target substance analog and the ligand (for example, the antigen-antibody reaction) with the naked eye or using a sensor. In addition, such a labeling substance may be used by connecting a target substance analog that can compete with the target substance to be analyzed, that is, a labeling substance that can specifically bind to a ligand.

As used herein, the term "conjugate" refers to a conjugate formed by connecting a labeling substance and the target substance analog to each other, and the first conjugate includes a labeling substance for signal detection and a target substance analog that competes with the target substance in the sample. A "second conjugate" refers to a conjugate formed by connecting a predetermined substance (or an arbitrarily injected predetermined substance) in a sample and a labeling substance to each other.

The labeling substance and the predetermined substance may be physically or chemically connected. That is, the labeling substance and the target substance analog may be connected by passive adsorption, and the labeling substance is modified to have a reactive group so that the labeling substance may be connected to a ligand by a covalent bond, but is not limited thereto, and the labeling substance and the target substance analog (or a predetermined substance in the sample) may be connected to each other using a method known to those skilled in the art.

However, the labeling substance and the target substance analog are connected in the form of the first conjugate before the sample is developed on the immunochromatographic strip, and are present in the conjugate pad in a separated state without being connected to each other, and they may be connected to each other to form a first conjugate while the sample is developed. In either case, the labeling substance and the target substance analog may move to the detection pad in a state of the first conjugate when the sample is developed, and this is because they are not immobilized on the conjugate pad.

According to an exemplary embodiment of the present invention, the labeling substance may include at least one selected from the group consisting of colloidal gold, latex particles, colored polystyrene microparticles, enzymes, fluorescent dyes, conductive polymers, luminous substances and magnetic particles.

Specifically, as used herein, the term "labeling substance" refers to a substance that generates a signal capable of being sensed with the naked eye or using a sensor. As the labeling substance, colloidal gold (gold particles), latex particles, colored polystyrene microparticles, enzymes, fluorescent dyes, conductive polymers, luminous substances, magnetic particles and the like may be used, but are not limited thereto.

Furthermore, the signal may be generated by itself due to the inherent characteristics of the labeling substance such as luminescence, or may be generated by external stimulation such as fluorescence.

According to another exemplary embodiment of the present invention, provided is an immunochromatographic strip, in which the first ligand, ligand 2-1 and ligand 2-2 include any one selected from the group consisting of a protein, an antigen, an antibody, DNA, RNA, PNA and an aptamer, and the first ligand includes a ligand different from ligand 2-1 and ligand 2-2.

As used herein, the term "ligand" refers to a substance that specifically binds to an antigen, a receptor, and the like. For example, an antibody that specifically binds to an antigen, a ligand that specifically binds to a specific receptor, and the like act as ligands. Non-limiting examples of the ligand include a protein, an antigen, an antibody, DNA, RNA, PNA or an aptamer, and in the present invention, the ligand can be used without limitation as long as it is a substance exhibiting the abovedefined properties. The meaning of the ligand used throughout the present specification means a substance that specifically binds to the materials as defined above, unless specifically described as a ligand that specifically binds to a particular receptor.

In a preferable exemplary embodiment of the invention, the labeling substance and the target substance analog may be linked in advance and present in the conjugate pad as the first conjugate. In this case, a conjugate solution is prepared by preparing each of the labeling substance and the target substance analog as a solution having a known concentration and mixing the solutions, and then allowing a reaction for a certain period of time, and may be aliquoted into the conjugate pad to prepare a conjugate pad provided with a conjugate. In this case, the concentration and mixing ratio of each solution may be determined in consideration of the binding ratio between the labeling substance and the target substance analog. For example, the binding between the labeling substance and the target substance analog may be a case where one target substance analog is bound to one labeling substance, a case where a plurality of target substance analogs are bound to one labeling substance, or a case where a plurality of labeling substances are bound to one target substance analog. The specific binding ratio is not important, but a preferred aspect may be to maintain a constant ratio. The binding ratio may be changed depending on the types of the labeling substance and the target substance analog, and may be predicted in consideration of relative sizes thereof, the number of binding sites, and the like. For example, when a latex bead with a size of several microns is used as a labeling substance and an antibody is used as a target substance analog, a plurality of antibodies may bind to one latex bead. Preferably, the concentration and mixing ratio of the antibody and the latex bead solution may be adjusted such that the antibody is saturated and bound to the latex bead surface in order to bind the antibody to each latex bead surface in a uniform number, but are not limited thereto.

As described above, a first conjugate solution having a known concentration may be obtained by mixing and reacting the labeled substance and the target substance analog, wherein each has a known concentration. The first conjugate solution is in the form of a dispersion in which a constant concentration of first conjugate molecules are uniformly dispersed in the solution.

Referring to FIG. 3, the immunochromatographic strip of the present invention is an immunochromatographic strip including one test line (T) immobilized using a ligand that specifically binds to a target substance or a target substance analog, and may include a detection pad provided with one test line (T) and two control lines (C1, C2) separated from each other.

According to still another exemplary embodiment of the present invention, it is possible to provide an immunochromatographic strip, in which the first conjugate in a mobile phase and the target substance in the sample competitively react with a first ligand of a test line, a first complex may be formed when the first conjugate reacts with the first ligand of the test line, and the number of first complexes is decreased as the concentration of the target substance in the sample is increased. Further, in the immunochromatographic strip of the present invention, the signal intensity of the test line may be decreased as the concentration of the target substance in the sample is increased.

The present invention forms a "direct competitive immune response test line" by immobilizing one test line on a detection pad such that not only the target substance in the sample can be qualitatively detected but also quantitatively analyzed. The second ligand of the control line of the immunochromatographic strip of the present invention does not react with the target substance, the target substance analog and the first conjugate in the mobile phase developed on the detection pad, and may be used by being immobilized on the detection pad.

In the present invention, the first ligand refers to a ligand that is immobilized on the test line of the detection pad to cause a reaction with the target substance, the target substance analog or the first conjugate in the sample.

In the present invention, the second ligand refers to a ligand that is immobilized on the detection pad to form a control line, and is different from the first ligand.

The first ligand, ligand 2-1 and ligand 2-2 adjust the signal intensity using only a predetermined specific amount, adjust the concentration range of the target substance in the sample, which can be detected through comparison with the signal intensity of a test line which shows changes depending on the concentration, and furthermore, may be helpful for a very reliable quantitative analysis.

A first ligand that reacts with a certain amount of target substance, target substance analog or first conjugate of the conjugate pad is immobilized on the test line of the immunochromatographic strip of the present invention, and the two control lines may be immobilized by varying or fixing the amounts thereof. Since the first ligand may specifically bind to the target substance immobilized on the conjugate pad, when the target substance is not present in the sample, the first conjugate is developed together with the mobile phase and binds to the test line on which the first ligand is immobilized in the detection pad to form a first conjugate-first ligand complex (first complex), thereby showing a strong signal. When the target substance is present in the sample, the target substance and the first conjugate in the sample are together developed into the mobile phase by the detection pad, and in this case, depending on the amount of target substance, the amount forming the first conjugate-first ligand complex of the test line is reduced, and accordingly, the signal is reduced. That is, the signal of the test line is reduced proportionally depending on the amount of target substance in the sample.

The second conjugate accommodated in the conjugate pad does not react with the first ligand, and reacts (binds to) with ligand 2-1 and ligand 2-2 of the control line immobilized on the detection pad to show a signal.

Quantitative analysis may be performed in a wide range of the target substances by comparing the ratio of the test line signal appearing depending on the concentration of the target substance in the sample and the control line signal. For example, it is possible to provide an immunochromatographic analysis method, including the steps of: allowing the first conjugate to bind to the first ligand to form a first complex and allowing the formed first complex to show a predetermined first signal according to a labeling substance; allowing the mobile phase to be developed into a first control line on which ligand 2-1 is immobilized and a second control line on which ligand 2-2 is immobilized; showing a predetermined second signal in the first control line and the second control line by allowing a second conjugate to bind to ligand 2-1 and ligand 2-2 whose concentrations are different from each other; and analyzing the amount of the target substance in the sample according to the first signal and the second signal.

As described above, immunochromatography uses the principle of chromatography in which a mobile phase including a target substance moves along a medium. Therefore, for immunoassay using the immunochromatographic strip, a mobile phase for moving the sample containing the target substance along the strip is required. Accordingly, the immunoassay kit of the present invention may additionally include a buffer solution. The buffer solution not only acts as a mobile phase to move the sample along the immunochromatographic strip, but also can act as a solvent to dissolve the conjugate, and may also serve as a diluent for diluting the sample. For example, when whole blood analysis is performed, a component for lysing a hemocyte component such as red blood cells may be additionally included. As the buffer solution, a typical buffer solution such as a 100 mM to 1 M sodium carbonate buffer solution, a non-ionic or amphoteric surfactant or a mixture thereof may be used without limitation, and the composition and usage ratio of the buffer solution may be appropriately selected depending on the type of a desired reaction, such as an antigen-antibody reaction.

The conjugate pad is a pad including a target substance analog that binds to the first ligand competitively with the target substance as described above and the labeling substance, and the target substance analog and the labeling substance may be connected in the form of a first conjugate and present in the conjugate pad even before the sample is developed, or may be present in the conjugate pad in a separated state without being connected to each other, and they may be connected to each other to form a conjugate while the sample is developed.

In the present invention, in the case of the first conjugate present in the conjugate pad, only a predetermined specific amount that has already been determined may be present. That is, the amount of the first conjugate means a specific amount of the target substance analog present in the conjugate pad. Since the amount of first conjugate in the conjugate pad is set to a certain amount, as the concentration of the target substance in the sample is increased, the first conjugate and the target substance compete for binding with the first ligand of the test line, and as the concentration of the target substance is increased, the amount of binding between the first conjugate and a target substance-specific ligand (first ligand) of the test line is decreased. In contrast, when the concentration of the target substance in the sample is low, an inversely proportional relationship in which the amount of binding between the first conjugate and the target substance-specific ligand (first ligand) of the test line is increased may be exhibited.

Preferably, the conjugate pad may be present in the pad in a conjugated state in which the target substance analog and the labeling substance are already connected. In this case, as described above, the conjugate pad may be prepared by applying a conjugate solution having a known concentration to the pad.

Furthermore, the conjugate pad may additionally include a second conjugate in which the specific detection substance and the labeling substance are bound such that it may be used as an internal control. The second conjugate may react specifically or non-specifically with the second ligand of the control line. The second conjugate may be captured by ligand 2-1 or ligand 2-2 of the control line to confirm whether the sample is developed or not. A more specific description of the control line and the second ligand will be described below.

The detection pad is a medium in which the mobile phase and the sample are developed, and the mobile phase and the sample can move by the capillary action of a porous membrane forming the detection pad. Furthermore, the detection pad is provided with the test line and the control line separated from each other. One end of the detection pad may be connected to the conjugate pad, and the other end may be connected to an absorbent pad that provides a driving force for sample transfer. The conjugate pad and the absorbent pad may be positioned to partially overlap the detection pad. The detection pad may include a porous membrane, and as the porous membrane, it is possible to use a nitrocellulose membrane, a glass fiber membrane, a polyethersulfone (PES) membrane, a cellulose membrane, a nylon membrane and a combination thereof, and preferably, a nitrocellulose membrane having pores with a size of 5 to 15 µm may be used, but the porous membrane is not limited thereto.

Hereinafter, the principle of qualitative and quantitative analysis using the immunochromatographic strip of the present invention will be more specifically described.

In immunochromatographic strips that use competitive reactions in the related art, a test line is formed by immobilizing a substance that is the same as the target substance or an analog thereof on the detection pad, and when there is no target substance in the mobile phase including the sample to be developed, the first ligand-labeling substance is bound to and captured by the target substance of the test line to generate a signal. When there is a target substance in the sample, the first ligand and the target substance react specifically to produce a complex of target substance-first ligand-labeling substance, the complex is developed along the mobile phase, and the amount of complex formed with the target substance of the test line is reduced depending on the amount of target substance-first ligand-labeling substance included in the mobile phase (competitive reaction). In this case, the signal intensity is reduced as the concentration of the target substance in the sample is increased. In this case, during the reaction between the first ligand and the target substance of the test line, when a small concentration of the target substance is present in the sample due to the strong specific binding force, even binding of the target substance to the first ligand in the sample does not affect the formation of the complex of the target substance-first ligand-labeling substance of the test line, and since there is almost no change when the color development of the test line is confirmed with the naked eye or using a sensor, a competitive reaction occurs only when the target substance is present at a certain high concentration in the sample, and when a high concentration of target substance reacts, the saturation point of the color development of the test line is avoided, and sensitivity is lowered, so that the range of the quantitative analysis is narrowed, and there are great restrictions on the quantitative analysis of the target substance.

However, in the present invention, although there is no difference in using a single test line in an immunochromatographic strip that uses a general competitive reaction, what is immobilized on the test line is a ligand (first ligand) that specifically binds to the target substance (or the target substance analog and the first conjugate), a method that causes a direct competitive immune response between the target substance in the sample and the first conjugate is used to enable analysis even at high concentrations of the target substance in the sample to improve sensitivity, and the present invention was devised such that quantitative analysis could be performed in a wide concentration range.

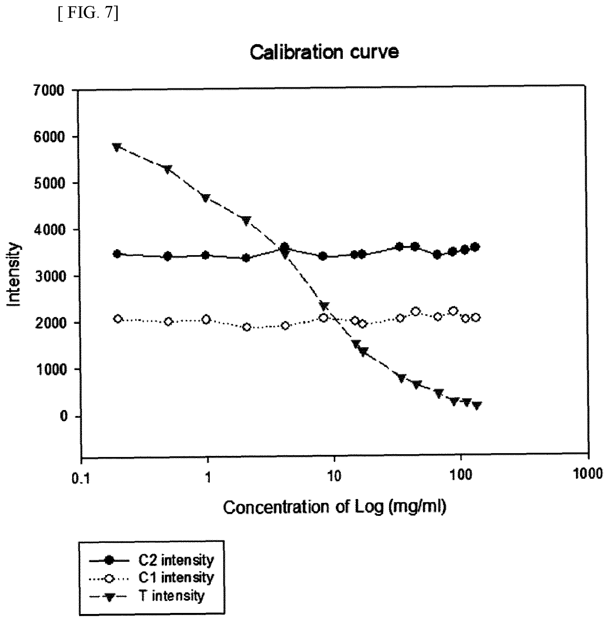

Referring to FIGS. 3 to 7, one test line and two control lines may be used, and when numbers are given to the test line and control lines in order of proximity to the conjugate pad, a quantitative graph may be obtained for the competitive reaction according to the concentration of the target substance in the sample as a ratio of the first control line (C1) or the second control line (C2) with respect to test line 1. Therefore, since it is possible to obtain a linear graph up to the high concentration range of the target substance in the sample, the sensitivity of the target substance may be improved and the quantitative measurement range (dynamic range) may be broadened from low concentration to high concentration (FIG 7). Since the test line conjugate pad is devised and applied to one strip such that the target substance and the first conjugate in the sample competitively react with a predetermined amount of first ligand and the concentration of the target substance can be quantitatively analyzed, it is characterized by its rapidity, ease of use, and the possibility of improved quantitative analysis with only one dilution without, particularly, a high dilution factor of a high concentration of target substance compared to immunochromatographic analysis methods in the related art.

According to an exemplary embodiment of the present invention, ligand 2-1 may be immobilized on the first control line, ligand 2-2 may be immobilized on the second control line, and ligand 2-1 and ligand 2-2 may bind to a second conjugate in which a predetermined substance different from the target substance is bound to the labeling substance. The concentration of ligand 2-1 immobilized on the first control line may be more than 0.5 to 1.0 mg/ml, and the concentration of ligand 2-2 immobilized on the second control line may be 0.1 to 0.5 mg/ml. Since ligand 2-1 and ligand 2-2 have different amounts of second conjugate bound at different concentrations on each control line, the intensity of the signal appearing due to the binding of the second conjugate is different, and thus may be used for quantitative analysis of the target substance.

Specifically, when a liquid sample is introduced into the sample pad of the strip of the present invention, the mobile phase and the sample begin to be developed. In the conjugate pad, a certain amount of first conjugate reacts competitively with the target substance in the sample. The introduced sample and a certain amount of first conjugate are developed along with the mobile phase. Here, since the amount of the first conjugate is constant, it is characterized in that the number of first conjugates bound to the first ligand immobilized on the test line is decreased as the concentration of the target substance in the sample is increased, and conversely, as the concentration of the target substance in the sample is decreased, the number of first conjugates bound to the first ligand immobilized on the test line is increased.

Since all of the first conjugates include a labeling substance, the presence or absence, amount, or both of the target substance in the sample may be measured by measuring the signal from the labeling substance in the first conjugate captured by the test line among the first conjugates.

As described above, as the concentration of the target substance in the sample is increased, the amount of the first conjugate binding to the test line is decreased, and conversely, the binding amount of the first conjugate is increased as the concentration of the target substance in the sample is decreased. Therefore, the signal intensity from the test line on which the conjugate is captured shows a pattern of decrease as the concentration of the target substance in the sample is increased. In addition, when one test line and two control lines are used as illustrated in FIG. 2, the control line develops color and a signal appears due to the binding of the first conjugate to the first ligand of the test line, and the signal changes and appears depending on the amount of target substance in the sample. The second conjugate may be captured by the first control line with a low concentration of ligand 2-1 and the second control line with a high concentration of ligand 2-2 to develop color, and the degree of color development may vary depending on the concentrations of ligand 2-1 and ligand 2-2. Therefore, since the degree of color development of the test line can be quantified using an accurate quantitative graph according to the wide concentration range with one experiment by comparison and evaluation of the degree of color development according to the amount of target substance in the sample even with the naked eye, it is possible to improve the sensitivity of the target substance and expand the quantitative measurement test range (dynamic range) of the linearity of the high-concentration target substance in the sample.

As a result, the target substance can be qualitatively and quantitatively analyzed through the signal from the labeling substance of the first conjugate captured by the test line where the competitive reaction occurs. More specifically, the concentration of the target substance may be analyzed by comparing the signal of the test line with the standard signal data previously completed for the target substance having a known concentration. The intensity of the signal may be visually confirmed and analyzed semi-quantitatively, or may be analyzed more precisely and quantitatively using a reader such as a densitometer.

As used herein, the term "control line" refers to a part which emits a constant signal regardless of the concentration of the sample or target substance in the sample. The invariant control line may be formed using a method similar to that of the test line. However, the control line may be formed by immobilizing a second ligand that can bind to the second conjugate that moves along the detection pad by the mobile phase together with the sample without binding to a substance to be analyzed (target substance). Alternatively, the control line may be formed by immobilizing a ligand (second ligand) that does not bind to a separate substance (first conjugate) in which a labeling substance or a labeling substance analog is bound while moving along the detection pad by the mobile phase together with the sample. As a result, the control line is formed by immobilizing a substance capable of emitting a constant signal regardless of the concentration and presence/absence of the target substance in the sample with a ligand (second ligand). As the second ligand that can be used as the control line, anti-rabbit IgG, anti-chicken IgY, streptavidin, bovine serum albumin, and the like may be used.

When a specific concentration of the second ligand is immobilized on the control line, the intensity of a signal generated here is always constant, so that the signal intensity on the test line is compared with the signal intensity on the control line and expressed as a ratio to more easily understand the signal intensity on the test line, and it is possible to determine to what extent the intensity of the test line is generated when the concentration of the target substance is at a certain level. The signal intensity of the control line may be compared with the signal intensity of the test line and used for quantitative analysis. That is, the ratio of the signal intensities of each of the control lines and the test line may be utilized as internal standard signal data.

For example, as illustrated in FIG. 6, when it was confirmed that when one test line was formed and a test line (T) for control line 1 (C1) at a constant concentration showed 2.5-fold, 1.5-fold and 1.0-fold signals through a target substance having a known concentration, the ratio of the concentration for control line 2 (C2) showed 1.5-fold, 1.0-fold and 0.5-fold signals, respectively, and as a result of analyzing a target substance having an unknown concentration, it can be seen that the test line (T) emits low, medium and high concentration signal intensities for control lines 1 (C1) and 2 (C2), and thus the concentration is between 5 to 140 mg/ml. The concentration may be easily checked with the naked eye by not only comparing the signal intensities of the test line and the control line to simple POCT medical equipment such as sensors, but also comparing the color development intensity of the control line.

The position and number of test lines and the position of the control lines using the competitive reaction of the target substance may be appropriately selected depending on the antigen-antibody reaction used and the like, and are not limited thereto. The presence or absence of the control line signal can be used to confirm whether or not the sample can be successfully developed, and the target substance can be quantitatively analyzed by comparing the signal intensity of the test line and the signal intensity of the control line with the already determined standard signal data. The standard signal data will be described below.

Specifically, the present invention provides an immunochromatographic strip in which a concentration measurement range (dynamic range) of a target substance in a sample is 5 mg/ml to 140 mg/ml.

In the present invention, in the case of quantitative analysis through the signal intensity of one immobilized test line and the signal intensity of each control line, the concentration measurement range (dynamic range) of the target substance in the sample may be between 5 mg/ml and 140 mg/ml. The immunochromatographic strip of the present invention is characterized by enabling a quantitative measurement, which was limited by only one test line and one control line in the related art and expanding the concentration measurement range and improving sensitivity to enable a user to easily and conveniently analyze the results because a plurality of suitable control lines are used for low concentration and high concentration that cannot be analyzed in a common single test line through the direct competitive immune response of the target substance with only one test line and one dilution from a sample containing a high concentration of target substance.

The immunochromatographic strip of the present invention will be more specifically described. The immunochromatographic strip may include a sample pad into which a sample for confirming whether a target substance is included is introduced; a conjugate pad whose one end is connected to the sample pad; a detection pad whose one end is connected to the other end of the conjugate pad; an absorbent pad whose one end is connected to the other end of the detection pad and which provides a driving force for sample transfer from the sample pad; and a solid support located at the lower portion of the immunochromatographic strip. The configurational view for the immunochromatographic strip of the present invention is illustrated in FIG. 2.

The solid support may be formed of a material selected from the group consisting of nitrocellulose, nylon, PVDF, glass and plastic. By attaching the strip to the solid support and manufacturing the strip, the durability of the strip may be enhanced, and handling and storage may be facilitated. Furthermore, the mounting of an additional external case may be facilitated. As a plastic material that may be used as the solid support, a polypropylene film, a polyester film, a polycarbonate film, an acrylic film and the like may be used, but the plastic material is not limited thereto.

According to another exemplary embodiment of the present invention, it is possible to provide a diagnostic kit in which an immunochromatographic strip is fixed in a case, wherein the case includes an upper case and a lower case, the lower case includes a strip support, and the upper case includes a sample inlet and a result confirmation window at a position corresponding to a test line or a control line. Further, it is possible to provide a diagnostic kit in which the support includes at least one selected from the group consisting of nitrocellulose, nylon, PVDF, glass and plastic.

As one aspect, the present invention may provide a diagnostic kit in which the immunochromatographic strip is additionally fixed in a case, wherein the lower case is provided with a guide and a strip support, and the upper case is provided with a result confirmation window at a position corresponding to a sample inlet, a test line and a control line.

The immunochromatographic strip may be additionally fixed in the case. The inside of the lower case may be provided with a plurality of guides and/or strip supports for disposing and fixing or compressing the immunochromatographic strip in an appropriate position. Optionally, the upper case may also be provided with a guide and a strip support at a position corresponding to the guide and the strip support provided in the lower case. That is, the guide and/or the strip support may be formed in the lower case or in both the upper and lower cases, if desired. Further, the upper case may be provided with a result confirmation window for detecting a signal from the labeling substance at a position corresponding to the sample inlet, the test line and the control line. The sample inlet may be formed in the form of a hole or slit, and the like at a point that is an end opposite to one end of the detection end, that is, an absorbent pad based on the test pad, and is sufficiently separated from a test line such that the sample may be simultaneously developed along the membrane. The result confirmation window may be formed to be large enough to be identified from the outside while including the test line and even a plurality of control lines on the detection pad. As long as the test line and a plurality of control lines can be confirmed, the size and shape thereof may be formed without limitation.

The upper and lower cases may be manufactured using a typical plastic material, and a material such as polycarbonate and acrylonitrile butadiene styrene (ABS) may be used, but is not limited thereto. The upper and lower cases may be separately manufactured and provided with a coupling groove, a coupling protrusion, or the like to be coupled by a typical means, and may be manufactured as an integrated type in some cases.

Furthermore, the diagnostic kit may be additionally provided with standard signal data including a color reference table of a test line and a plurality of control lines for samples including a target substance having various known concentrations.

As used herein, the standard signal data refers to data that summarizes the signal intensity in one test line and two control lines using the immunochromatographic strip of the present invention for the samples including a target substance having various known concentrations. Such standard signal data can be summarized in various forms, representatively by the colors of the two control lines and the test line corresponding to the concentration of each target substance. Similarly, in the case of litmus test paper for measuring pH, there is an example of providing a color standard table in which the colors of the test paper corresponding to each pH are summarized. In particular, in the case of the standard signal data including the color reference table, the intensity of the signal for the target substance may be semi-quantitatively analyzed conveniently and rapidly because the signal intensity may be easily compared with the standard signal data with the naked eye. In this regard, the diagnostic kit may be able to perform qualitative or semi-quantitative analysis by visually confirming the presence or absence and intensity of the signal of the labeling substance from the test line and the two control lines. That is, by visually comparing the standard signal data including the color reference table that may be previously provided with the presence or absence and intensity of a signal appearing on the test line and the two control lines after developing the sample into the kit, the presence or absence and concentration of the target substance in the sample may be analyzed.

According to another exemplary embodiment of the present invention, provided is an immunochromatographic analysis method including the steps of: injecting a predetermined liquid including a sample into a sample inlet; forming a mobile phase by accommodating the liquid in a sample pad; allowing the mobile phase to be developed into a conjugate pad and include a first conjugate; allowing the first conjugate and the target substance in the sample to competitively bind to a first ligand while the mobile phase including the first conjugate is developed into a test line; allowing the first conjugate to bind to the first ligand to form a first complex and allowing the formed first complex to show a signal according to a labeling substance; and analyzing the amount of the target substance in the sample according to the signal.

Further, provided is an immunochromatographic analysis method further including: showing a separate signal by allowing the mobile phase to pass through the control line and allowing a predetermined substance to bind to a second ligand; and analyzing the amount of target substance in the sample by comparing a signal shown on the test line with a signal shown on the control line.

As another aspect, the present invention provides a method for qualitatively or quantitatively analyzing a target substance in a sample using the immunochromatographic strip, the method including: a first step of introducing and developing a sample into the conjugate pad or a pad located before the conjugate pad; a second step of confirming the presence or absence and intensity of a signal of a labeling substance from one test line and two control lines; and a third step of measuring the amount of target substance by comparing the signal intensity confirmed from the one test line and the two control lines with standard signal data, wherein the standard signal data is obtained by performing the first step and the second step, respectively, for samples including a target substance having various known concentrations.

The specific principle for the method of qualitative and/or quantitative analysis of the target substance in the sample using the immunochromatographic strip of the present invention is as described above. Furthermore, the standard signal data is also as described above.

In carrying out a specific analysis method, the method be performed in the following order. First, a liquid sample including a substance to be analyzed (target substance) may be put into the conjugate pad or a pad located before the conjugate pad. That is, the liquid sample may be introduced into the strip by putting the sample directly into the conjugate pad, but preferably, the sample may be introduced before the conjugate pad by putting the sample, for example, into the sample pad. Further, the sample may be uniformly mixed by adding a buffer solution such as phosphate buffer (PB), and may be introduced into the strip by the same method.

When the sample is loaded (introduced) into the immunochromatographic strip as described above, the development begins, and then it can be confirmed through the control line whether the sample is successfully developed. When the sample is successfully developed, the presence or absence and intensity of the labeling substance signal from the test line and the two control lines are confirmed. When a change in the signal is confirmed from the test line, this indicates that the target substance is included in the sample (qualitative analysis). Furthermore, by comparing the signal intensities confirmed from one test line and two control lines with the standard signal data, it can be confirmed that the target substance is included in the sample at a certain concentration (quantitative analysis).

In specific exemplary embodiments of the present invention, by analyzing bovine IgG (target substance) using an immunochromatographic strip additionally including a control line, it was confirmed that a very reliable quantitative analysis can be made with the naked eye when the concentration of the target substance in the sample is in a range of 5 to 140 mg/ml (FIG 6).

Samples for the immunoassay of the present invention include all biological samples from mammals, such as, preferably, whole blood, serum, plasma, bone marrow fluid, sweat, urine, tears, saliva, skin, mucous membranes, and hair isolated from living organisms, and may be, for example, colostrum isolated from a cow. As blood, serum or plasma components from which hemocyte components have been removed may be used, and when whole blood is used, components capable of lysing hemocyte components may be added to a buffer solution and used. This is exemplary, and a sample for immunoassay of the present invention is not particularly limited.

The immunoassay method according to the present invention may be useful for semi-quantitative or quantitative diagnosis of various drugs such as proteins that are small in size or can use only one binding site such as peptide proteins, vitamin D, immunoglobulins, allergens, and antibiotics.

Hereinafter, the present invention will be described in more detail through Examples. These Examples are provided only for more specifically describing the present invention, and the scope of the present invention is not limited by these Examples.

### [Example 1]

### Manufacture of immunochromatographic dip strip

### A. Manufacture of detection pad in which test line and invariant control line are formed

Three analysis lines were aliquoted into a nitrocellulose membrane. The nitrocellulose membrane was laminated on a plastic card using a laminator. Thereafter, an anti-bIgG antibody (anti-bovine immunoglobulin G, USA) that detects a target substance or an analog thereof and anti-chicken immunoglobulin (IgY, USA) as a second ligand were aliquoted at constant intervals at the position of test line 1 and the positions of control lines 1 and 2, respectively, using an automatic dispenser, and then dried at 20 to 25°C for 24 hours.

### B. Manufacture of conjugate pad

A conjugate pad was used as it was without any treatment in a state where moisture was completely dried in a dryer, and was prepared by cutting the conjugate pad into an appropriate size. Polyester (polyester pad) was used as it was as a conjugate pad.

A conjugate solution in which colloidal gold particles with a diameter of about 20 nm and bovine IgG from cow (Equitech, USA) were bound was prepared, and further, a second conjugate solution in which colloidal gold particles having a diameter of about 20 nm and chicken IgY (Arista, USA) were bound was prepared. The conjugate solution and the second conjugate solution were aliquoted into a conjugate pad, completely dried, and then prepared by cutting the conjugate pad into an appropriate size.

### C. Manufacture of sample pad

A sample pad was used as it was without any treatment in a state where moisture was completely dried in a dryer, and was prepared by cutting the conjugate pad into an appropriate size.

### D. Manufacture of absorbent pad

An absorbent pad was used as it was without any treatment in a state where moisture was completely dried in a dryer, and was prepared by cutting the absorbent pad into an appropriate size.

### E. Manufacture of immunochromatographic strip

The detection pad, conjugate pad, sample pad and absorbent pad prepared through each of the above processes were assembled according to the structure illustrated in FIG. 2

That is, the sample pad was attached so as to overlap one end of the conjugate pad, one end of the detection pad was attached so as to overlap the other end of the conjugate pad, and the other end of the detection pad and one end of the absorbent pad to which a sticker for displaying the upper end was attached were attached so as to overlap each other. The strip illustrated in FIG 2 was manufactured by cutting the assembly into approximately 4 ± 1.0 mm using a cutter.

### [Example 2]

### Immunoassay using immunochromatographic dip strip

100 µl each of a sample solution including bovine immunoglobulin G (IgG) as a target substance and a sodium carbonate buffer solution was aliquoted into a 96-well plate. The sample solution was prepared such that the concentration of bovine immunoglobulin G (bIgG) was 2, 5, 10, 20, 40, 80, 100, 142, and 213 mg/ml, respectively.

The immunochromatographic strip manufactured in Example 1 was placed in a 96-well plate including the sample solution, and then developed for 10 minutes.

FIG. 6 is an actual strip photograph illustrating the development results of the chromatographic strip according to the concentration of the bovine immunoglobulin G, which is a target substance in colostrum.

For test line 1, it was confirmed that the signal intensity varies depending on the concentration of the target substance, and it was confirmed that it is possible to obtain a linear quantitative curve depending on the concentration ratio of control lines 1 and 2.

Furthermore, it can be confirmed that the concentration measurement range (dynamic range) for the target substance of the immunochromatographic strip appears widely from 5 mg/ml or less to 140 mg/ml or more.

According to an exemplary embodiment of the present invention, the immunochromatographic strip of the present invention can include a test line which uses a direct competitive immunoassay reaction to overcome difficulty in quantitatively measuring a high concentration of target substance and rapidly and conveniently perform not only a qualitative analysis with the naked eye, but also a very reliable quantitative analysis. Furthermore, the immunochromatographic strip of the present invention has an advantage in that the sensitivity and concentration measurement range of the target substance are improved using one test line and two control lines, each with a different concentration ratio, and furthermore, there is an advantage in that a user can understand the results easily and rapidly.

According to another exemplary embodiment of the present invention, the volume of the target substance in the sample can be easily measured using a diagnostic kit including the immunochromatographic strip.

According to still another exemplary embodiment of the present invention, provided is an immunochromatographic analysis method having high sensitivity when the volume of the target substance is measured.

Unless otherwise defined, all the terms (including technical and scientific terms) used in the present specification may be used as a meaning which may be commonly understood by a person with ordinary skill in the technical field to which the present invention pertains. In addition, the terms defined in generally used dictionaries are not to be interpreted ideally or excessively unless clearly and specifically defined. The terms used in the present specification are used merely to describe embodiments, and are not intended to limit the present invention. In the present specification, the singular forms include the plural forms unless otherwise specified in the phrases.

As described above, the embodiments of the present invention have been described, but it will be understood by a person with ordinary skill in the art to which the present invention pertains that the present invention can be implemented in other concrete forms without changing the technical spirit or essential features of the present invention. Therefore, it should be understood that the above-described examples are only illustrative in all aspects and not restrictive.

## Claims

1. An immunochromatographic strip, comprising: a sample pad for accommodating a liquid sample; a conjugate pad connected to one side of the sample pad and accommodating a labeling substance and a target substance analog; a detection pad connected to one side of the conjugate pad and comprising a test line, a first control line, and a second control line; and an absorbent pad connected to one side of the detection pad and receiving liquid remaining after a reaction, wherein the test line comprises a first ligand that optionally reacts with the target substance and a first conjugate, the first control line comprises ligand 2-1 that reacts with the second conjugate, and the second control line comprises ligand 2-2 reacting with the second conjugate, wherein the concentrations of ligand 2-1 and ligand 2-2 are different from each other.

2. The immunochromatographic strip of claim 1, wherein a concentration measurement range (dynamic range) of the target substance in the sample is 5 mg/ml to 140 mg/ml.

3. The immunochromatographic strip of claim 1, wherein the first conjugate consists of a combination of an analog of the target substance and a target substance, the first conjugate and the target substance in the sample competitively react with a first ligand of a test line, and a first complex is formed when the first conjugate reacts with the first ligand of the test line.

4. The immunochromatographic strip of claim 3, wherein the first complex is captured by a test line and shows a signal, and the amount of target substance in the sample is measured using the signal.

5. The immunochromatographic strip of claim 4, wherein the signal intensity of the test line is decreased as the concentration of the target substance in the sample is increased.

6. The immunochromatographic strip of claim 1, wherein the first ligand, ligand 2-1 and ligand 2-2 comprise any one selected from the group consisting of a protein, an antigen, an antibody, DNA, RNA, PNA and an aptamer, and the first ligand comprises a ligand different from ligand 2-1 and ligand 2-2.

7. The immunochromatographic strip of claim 1, wherein the labeling substance comprises at least one selected from the group consisting of colloidal gold, latex particles, colored polystyrene microparticles, enzymes, fluorescent dyes, conductive polymers, luminous substances and magnetic particles.

8. A diagnostic kit in which the immunochromatographic strip of any one of claims 1 to 7 is fixed in a case, wherein the case comprises an upper case and a lower case, the lower case comprises a strip support, and the upper case comprises a sample inlet; and a result confirmation window at a position corresponding to a first control line and a second control line.

9. The diagnostic kit of claim 8, wherein the support comprises at least one selected from the group consisting of nitrocellulose, nylon, PVDF, glass and plastic.

10. An immunochromatographic analysis method, comprising the steps of:
injecting a predetermined liquid comprising a sample into a sample inlet; forming a mobile phase by accommodating the liquid in a sample pad; allowing the mobile phase to be developed into a conjugate pad and comprise a first conjugate; allowing the first conjugate and the target substance in the sample to competitively bind to a first ligand while the mobile phase comprising the first conjugate is developed into a test line; allowing the first conjugate to bind to the first ligand to form a first complex and allowing the formed first complex to show a first signal according to a labeling substance; allowing the mobile phase to be developed into a first control line on which ligand 2-1 is immobilized and a second control line on which ligand 2-2 is immobilized; showing a second signal in the first control line and the second control line by allowing a second conjugate to bind to ligand 2-1 and ligand 2-2 whose concentrations are different from each other; and analyzing the amount of the target substance in the sample according to the first signal and the second signal.
